# EUROPEAN PATENT SPECIFICATION

(11) **EP 1 487 340 B1**
(45) Date of publication and mention of the grant of the patent: **23.01.2008**
(21) Application number: 02755408.8
(22) Date of filing: 10.07.2002
(51) Int. Cl.: A61B 5/15, A61B 5/00

(54) **SYSTEM FOR POINT OF CARE DIAGNOSIS AND/OR ANALYSIS**
DIAGNOSE- UND/ODER ANALYSESYSTEM AM BEHANDLUNGSORT
SYSTEME DE DIAGNOSTIC ET/OU D'ANALYSE SUR LE SITE D'ADMINISTRATION DES SOINS

(30) Priority: 10.07.2001 EP 01116592
(43) Date of publication of application: 22.12.2004
(73) Proprietor: Koninklijke Philips Electronics N.V., 5621 BA Eindhoven (NL)
(72) Inventor: BOOS, Andreas, NL-5656 AA Eindhoven (NL); BACHER, Johannes, NL-5656 AA Eindhoven (NL); PROSS, Gerhard, NL-5656 AA Eindhoven (NL); SEHER, Jens-Peter, NL-5656 AA Eindhoven (NL)
(74) Representative: Volmer, Georg
(86) International application number: PCT/IB2002/003004
(87) International publication number: WO 2003/005905

(56) References cited:
- WO-A-00/19887
- WO-A-01/28414
- US-A- 5 048 539
- US-A- 5 405 510

## Description

The present invention relates to a system for point of care diagnosis and/or analysis, and to a cartridge and to an apparatus thereof. Such a system could be a bench analyzer, a STAT lab or a POC diagnosis system.

Over the last years point of care diagnosis and analysis has become more and more accepted. With this technology, body fluids analysis and in particular blood analysis is performed at the patient bed side, e.g. in hospitals, outpatient centers or at home, within a few minutes without the need to send a sample of the body fluids to a central lab.

The system comprises a small cartridge having a sample receiving room or volume for receiving a sample of the body fluids to be analysed and/or diagnosed. For example a blood sample is injected into the cartridge which contains means for measuring the concentration of one or more specific components of the respective sample. In particular the measuring means comprise chemistry to detect the components, e.g. specific ions, proteins, antibodies etc, in the blood sample under test (some kind of a 'mini' lab). The cartridge further comprises electrical or optical connecting means at which an electric and/or optical value or signal is measurable correlating with the concentration of the respective components. This connecting means e.g. comprise electrodes exposed to the sample.

This cartridge will be connected to a diagnosis and/or analysis apparatus of the system measuring the respective concentration values or signals via said connecting means. For example the diagnosis and/or analysis apparatus is provided as a small analyzer (handheld or small benchtop) in which the cartridge can be inserted. This apparatus comprises means for diagnosing and/or analysing which e.g. qualify and quantify the chemical or biochemical reactions on the cartridges. The apparatus usually displays the measurement results, stores or prints them out and/or sends them to a central station or data management system.

The chemistry, process technology and measurement technique used for the above described cartridges unfortunately show large variations between manufacturing lots. To ensure high measurement reliability, accuracy and repeatability when determining the concentrations of the different substances, each manufacturing lot has to be characterised after production and lot specific calibration and characterisation data must be supplied together with the final cartridges. These calibration and characterisation data must be entered into the diagnosis and/or analysis apparatus which use these data to map the measured data to real concentration levels, i. e. the real concentration of the respective components will be determined in accordance to these data.

Different technologies are used today to transfer the calibration and characterisation data into the diagnosis and/or analysis apparatus. For example these data will be typed in as alphanumeric codes by hand, i. e. reading the codes from the cartridge package or the shipping containers and enter them into the apparatus by hand typing. This method is cheap but time consuming and very inconvenient for the medical staff. In addition it's a potential source for errors. Typing errors may lead to wrong measurement results and thus lead to critical safety problems.

It is also usual to provide a package, in which the cartridge is stored, with a barcode which will be scanned in with a barcode reader. This method requires a barcode reader as part of the apparatus. Problems may occur if the barcode on the cartridge package is illegible, e. g. not well printed, cracked, smeared, dirty etc. The user also needs to adjust the distance to the barcode reader and the scanning speed to get valid results.

Another solution prefers a memory chip, storing all required data. This memory chip will be placed into each shipping container. The chip must be plugged into the apparatus which then reads the calibration and characterisation data. This method does not allow to use more than one apparatus per shipping container, because per shipping container there is only one chip available. There exists also the danger that cartridges from different containers with different manufacturing lots may be mixed up resulting in wrong measurement results. Loosing the chip renders all the remaining cartridges of that shipping container unusable.

It is also possible to perform an individual'wet'calibration for each shipping container. Individual calibrations for each shipping container are performed by the end user by means of measuring and correcting the measurement results from well known reference samples. This method is very inconvenient and time consuming since the user must supply'gold standards'with adequate precision for each possible measurement. Forgetting to perform this tedious calibration will result in measurement errors.

US 5,405,510, for example, teaches a portable analyte measuring device for the measurement of fluid samples. The device has a disposable cartridge, a calibrating fluid external to the cartridge and an analyzer. The cartridge has a housing, inlet for introduction of fluids into the housing, flow cell in fluid connection with the inlet, and at least one flow control means in fluid connection with the reservoir. The flow cell has a flow-through channel with one or more hydrated sensors arranged along the channel that has fluid therein. The flow cell also has an electric circuit attached for electrical conduction to the sensors and reference electrode. The device also has signal conveyor connected to the electric circuit of the flow cell to transmit signals responsive to the fluids contacting the sensors to the analyzer. The cartridge and the calibrating fluid are associated with an encoded information carrier for sensor performance parameters and calibrant analyte concentrations for inputting into the encoded information reader of the analyzer.

It is therefore an object of the present invention to improve the correlation between a cartridge and cartridge specific data and/or information and to make the handling process easier. The object is solved by the independent claims. Preferred embodiments are shown by the dependent claims.

According to the invention each cartridge will be provided with storage means for storing cartridge specific data and/or information readable for the diagnosis and/or analysis apparatus. According to the invention the corresponding diagnosis and/or analysis apparatus will be provided with reading means for reading these cartridge specific data and/or information stored in the storage means of the cartridge. This diagnosis and/or analysis apparatus also will be provided with diagnosing and/or analysing means performing the measurement and/or the evaluation of the respective concentration values in accordance to the cartridge specific data and/or information. By adding permanent storage means to each individual cartridge, the cartridge itself contains all required data and/or information. The invention provides an inseparable combination between the cartridge and its specific data and/or information. It is now possible to provide mixed batches of different cartridges, i.e. cartridges of different manufacturing lots and/or of different type. Therefore the invention helps to avoid the drawbacks of all previously mentioned methods for data transfer.

Preferably the reading of the cartridge specific data and/or information will be performed automatically each time a cartridge is inserted into the apparatus. Therefore a manual interaction is no longer required. This leads to a reliable data transfer and time saving.

According to a preferred embodiment the cartridge specific data and/or information comprises at least one of the following data and/or information:
- Calibration and/or characterisation data relating to the respective cartridge and/or manufacturing lot data relating to the lot, to which the respective cartridge belongs. With this information wrong combinations of cartridges and characterising data will be avoided.
- Production date of the respective cartridge and/or shelf life information or expiration date of the respective cartridge. With this information it is possible to prevent usage of expired cartridges. This control previously had to be checked manually.
- Cartridge type information. There may be provided different types of cartridges provided for different diagnosis and/or analysis tasks. To perform a proper measurement or evaluation it is important for the apparatus to know the specific type of inserted cartridge. With this information wrong usage leading to measurement errors will be avoided.
- Unique serial number of the respective cartridge. With this information it will be possible to perform a reliable quality management tracking. In case of manufacturing errors it is also possible to suspend specific serial numbers to prevent usage of faulty cartridges.

Some types of cartridges require precise temperature measurements, e.g. gas concentrations are highly dependent on the sample temperature. According to another advantageous embodiment the cartridge will comprise means for temperature measurement, wherein an electrical value correlating with the temperature of the sample is measurable at the connecting means, wherein the cartridge specific data and/or information comprises calibration data of the temperature measurement means. Having a memory on the cartridge allows using low precision temperature measurement sensors on the cartridge in conjunction with stored calibration data, i.e. temperature measurement correction coefficients. Previously, the temperature measurement sensors, when part of the cartridge, have been expensive due to the need for high accuracy or selected versions.

According to a further embodiment the storage means will be provided to be writable and at least one of the following additional data and/or information will be storable or alterable in the storage means:
In case of a single use cartridge it will be stored, whether it is used or not. Therefore accidental reuse of a used single use cartridge can be prevented reliably.
In case of a multiple use cartridge it will be stored, how often and/or since when is in use. With this information counting errors and therefore inadmissible usage can be avoided.

The results of each diagnosis and/or analysis relating to the respective cartridge will be stored. It is therefore possible to transfer data sets to a data management system, e.g. by plugging the used cartridge into a special reader. Additionally it will be stored a patient identification information and/or an operator identification information and/or an identification information relating to the diagnosis and/or analysis apparatus and/or the date of the diagnosis and/or analysis. With this useful information there will be prevented a faulty mixing of the measurement results with the respective patient.

According to another embodiment there will be provided electric, electronic, magnetic and/or optical data transfer means for reading and/or writing and/or altering data and/or information stored in the storage means. These data transfer means support an automatic, time saving and error free data transfer between each cartridge and the apparatus.

Relating to a preferred embodiment the data transfer means comprise the respective connecting means of the cartridge and/or radio frequency transfer means. Using the connecting means leads to a relative simple design. Using radio frequency or optical transfer means has the advantage that the storage means can easily be read and/or programmed and/or altered without contact even when the cartridge is sealed in its package. E.g. it is possible to write on the storage means after the cartridge is sterilised. With radio frequency transfer means it is also possible to transmit the required power.

According to a special embodiment the storage means will comprise an electrical memory member, e.g. EEPROM, FRAM, PROM or battery backed RAM, readable and/or writable via electric or electronic data transfer means, or at least one magnetic stripe or an optical memory or a two dimensional barcode.

Other objects and many of the attendant advantages of the present invention will be readily appreciated and become better understood by reference to the following detailed description when considered in connection with the accompanying drawing. Features that are substantially or functionally equal or similar will be referred to with the same reference sign(s).

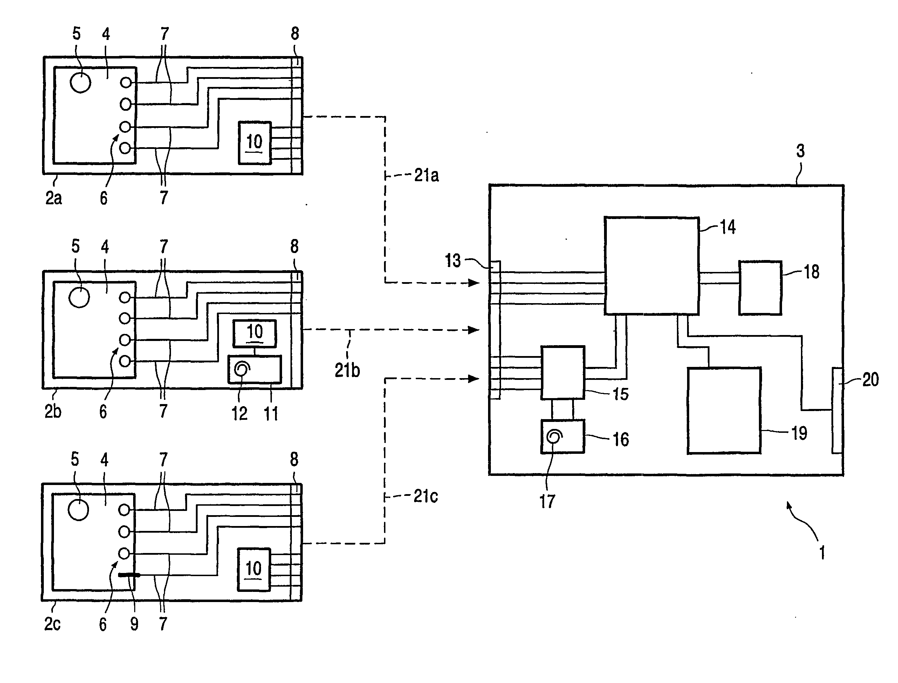

Fig. 1 depicts a schematic view of a system according to the invention.

Referring to Fig. 1 a system 1 for point of care diagnosis and/or analysis of body fluids of a patient according to the invention comprises at least one cartridge 2. In fig. 1 are shown three different embodiments of this cartridge 2 indicated with 2a, 2b and 2c. The system 1 also comprises at least one diagnosis and/or analysis apparatus 3.

Each cartridge 2 has a sample receiving room 4 to receive a sample of the body fluids, e.g. blood or urine, that has to be diagnosed and/or analysed. The cartridge 2 comprises a filler socket 5 through which the sample can be filled in the sample receiving room 4. Each cartridge 2 comprises measuring means 6 provided for measuring the concentration of at least one specific component or substance of the sample. These measuring means 6 may comprise chemistry performing specific chemical and/or biochemical reactions with the respective body fluids. The measuring means 6 also enclose e.g. electrodes 7 which are connected with electric connecting means 8 of the cartridge 2 and provide electrical values or signals correlating with the concentration of the respective components. These concentration values are therefore measurable at the connecting means 8.

In contrast to the cartridges 2a and 2b the cartridge 2c additionally comprises temperature measurement means 9 provided for measuring the temperature of the sample. These temperature measuring means 9 are also connected with the connecting means 8 to provide there electrical values or signals correlating with the temperature of the sample.

Each cartridge 2 comprises storage means 10 which can be provided as an electrical memory member, e.g. a memory chip, in particular EEPROM, FRAM, PROM or battery backed RAM. This storage means 10 are designed as permanent memory and are readable with appropriate reading means. It is also possible to use writable and alterable memory. In the cartridges 2a and 2c the storage means 10 are connected with the connecting means 8 of the respective cartridge 2.

Cartridge 2b shows a special embodiment having radio frequency data transfer means 11 symbolised with an antenna 12. These radio frequency data transfer means 11 communicate with the storage means 10 and will be provided additionally or instead of the connection with the connecting means 8.

In the storage means 10 are stored cartridge specific data and/or information, e.g. calibration and/or characterisation data relating to the respective cartridge 2, and/or manufacturing lot data relating to the lot, to which the respective cartridge 2 belongs, and/or a production date of the respective cartridge 2, and/or shelf life information or an expiration date of the respective cartridge 2, and/or cartridge type information, and/or a unique serial number of the respective cartridge 2. This list is only an example and is in particular not complete.

The diagnosis and/or analysis apparatus 3 comprises electrical connecting means 13 corresponding to the connecting means 8 of the cartridges 2. The apparatus 3, which can be a handheld or benchtop, further comprises diagnosing and/or analysing means 14, which can be represented by a programmed or programmable microprocessor. This diagnosing and/or analysing means 14 are connected with the connecting means 13 of the apparatus 3. The apparatus 3 also comprises reading means 15 connected with the connecting means 13. These reading means 15 are provided for reading the data and/or information stored in the storing means 10 of any cartridge 2 connected to the apparatus 3.

These reading means 15 additionally may be provided with radio frequency data transfer means 16 symbolised with an antenna 17. These radio frequency data transfer means 16 and the radio frequency data transfer means 11 of the cartridges 2 are provided to perform a communication between the storage means 10 of the respective cartridge 2 and the diagnosing and/or analysing means 14. Therefore the diagnosing and/or analysing means 14 can read in and/or write on the storage means 10 via the reading means 15.

The apparatus 3 also comprises a storage or memory member 18 storing e.g. coefficients and/or algorithms and/or parameters which will be required for evaluating the measured values or signals. There may be provided display means 19 to indicate the results of the diagnosis and/or analysis, and an interface 20 with which the apparatus 3 can communicate with peripheral equipment, e.g. a printer, or with a data management system.

The system 1 according to the invention works as follows:

Each manufacturing lot of cartridges 2 has its own lot data leading to specific calibration and characterisation data for the cartridges 2 of this lot. These specific calibration and characterisation data and preferably a number of other useful cartridge specific data and/or information are stored in the storage means 10 of each cartridge 2. Therefore every cartridge 2 is inseparably combined with this information. The storing of the data and/or information can be realised via the connecting means 8 or via the radio frequency data transfer means 11. The latter has the advantage of storing the data after sterilisation and packaging the cartridge 2.

After filling a sample of body fluids, e.g. a blood sample, into the sample receiving room 4, the respective cartridge 2 is inserted into the diagnosis and/or analysis apparatus 3 to provide a communication between the connecting means 8 of the cartridge 2 and the connecting means 13 of the apparatus 3. This insertion is indicated with broken lines 21a, 21b and 21c.

The reading means 15 can automatically read the stored cartridge specific data and/or information, especially the calibration and characterisation data of the inserted cartridge 2 via the connecting means 13 and/or via the radio frequency data transfer means 16. Since the cartridge specific data and/or information comprise cartridge type information, the diagnosing and/or analysing means 14 will select appropriate measurement and/or evaluation routines.

Via the connecting means 8 and 13 the diagnosing and/or analysing means 14 will measure the electric values correlating with the concentration of the specific components and - in case of cartridge 2b - also values correlating with the temperature of the sample. Before this measurement is performed or before an evaluation of this values takes place the diagnosing and/or analysing means 14 will perform some routines like the following:

According to the cartridge specific data and information the diagnosing and/or analysing means 14 will compare the expiration date of the connected cartridge 2 with the current date, and perform the measuring and/or evaluation of the concentration and/or temperature values, only if the expiration date is not exceeded.

Alternatively or additionally the diagnosing and/or analysing means 14 compare the serial number of the connected cartridge 2 with invalid serial numbers stored in the memory member 18 of the diagnosis and/or analysis apparatus 3, and perform the measuring and/or evaluation of the respective values, only if the serial number of the connected cartridge 2 is not invalid.

In case of a connected single use cartridge 2 the diagnosing and/or analysing means 14 alternatively or additionally check, whether this cartridge 2 is used or not and perform the measuring and/or evaluation of the respective values, only if the cartridge 2 is unused.

In case of a connected multiple use cartridge 2 the diagnosing and/or analysing means 14 alternatively or additionally check, how often and/or since when this cartridge 2 is used and perform the measuring and/or evaluating of the respective values, only if the cartridge 2 is still usable.

Since these routines have valid results the diagnosing and/or analysing means 14 will perform an evaluation of the measured values. This evaluation has to be performed with appropriate coefficients and/or parameters and/or algorithms for determining the real component concentration and the real sample temperature, respectively. In accordance with the automatically read calibration and characterisation data the diagnosing and/or analysing means 14 select the appropriate coefficients and/or parameters and/or algorithms which are stored in the memory member 18. In case of an evaluation which requires precise temperature measurements the diagnosing and/or analysing means 14 evaluate the respective concentration value in accordance to the probe temperature.

The results of this evaluation and/or administrative data can be stored in the storage means 10 of the respective cartridge 2, in particular in case of a multiple use cartridge 2. To this aim the apparatus 3 comprises writing means which preferably are incorporated in the reading means 15. There also may be provided processing means for controlling the data transfer, in particular storing and/or altering of data. This processing means preferably will be incorporated in the diagnosis and/or analysing means 14. E.g. in case of a single use cartridge 2 a used signal or stamp is stored on its storage means 10. In case of a multiple use cartridge 2 a usage counter is increased. For multiple use cartridges 2 it is also possible to store the results of each measurement together with the patient and/or operator identification codes and a time or date signal or stamp. Therefore a set of data is provided which finally can be transferred to a data management system, e.g. by plugging the used cartridge 2 into a special reader.

### LIST OF REFERENCES

- 1.: system for point of care diagnosis and/or analysis
- 2.: cartridge
- 3.: diagnosis and/or analysis apparatus
- 4.: sample receiving room
- 5.: filler socket
- 6.: concentration measuring means
- 7.: electrode
- 8.: connecting means of 2
- 9.: temperature measurement means
- 10.: storage means
- 11.: radio frequency transfer means of 2
- 12.: antenna of 11
- 13.: connecting means of 3
- 14.: diagnosing and/or analysing means
- 15.: reading means
- 16.: radio frequency transfer means of 3
- 17.: antenna of 16
- 18.: memory member of 3
- 19.: display means
- 20.: interface
- 21.: connecting between 2 and 3

## Claims

1. A cartridge of a system for point of care diagnosis and/or analysis of body fluids of a patient, comprising:
- a sample receiving room (4) for receiving a calibration fluid and/or a sample of the body fluids to be diagnosed and/or analysed,
- means (6) for measuring the concentration of at least one specific component of the sample,
- electric or optical connecting means (8), with which the cartridge (2) is connectable to a diagnosis and/or analysis apparatus (3), and at which an electric or optic value is measurable correlating with the concentration of the respective component,
**characterised by** storage means (10) for storing cartridge specific data and/or information readable for the diagnosis and/or analysis apparatus (3).

2. The cartridge of claim 1, wherein the cartridge specific data and/or information comprises at least one of the following data and/or information:
- calibration and/or characterisation data relating to the respective cartridge (2),
- manufacturing lot data relating to the lot, to which the respective cartridge (2) belongs,
- production date of the respective cartridge (2),
- shelf life information or expiry date of the respective cartridge (2),
- cartridge type information like single/multi use, number and type of measurement parameters,
- unique serial number of the respective cartridge (2).

3. The cartridge of claim 1 or 2, wherein the cartridge (2) comprises means (9) for temperature measurement, wherein an electrical value correlating with the temperature of the sample is measurable at the connecting means (8), wherein the cartridge specific data and/or information comprises calibration data of the temperature measurement means.

4. The cartridge according to any one of the claims 1 to 3, wherein the storage means (10) are provided to be writable and wherein at least one of the following additional data and/or information is storable or alterable in the storage means (10):
- in case of a single use cartridge (2) it is stored, whether it is used or not,
- in case of a multiple use cartridge (2) it is stored, how often and/or since when it is used,
- the results of each diagnosis and/or analysis relating to the respective cartridge (2) are stored, wherein a patient identification information and/or an operator identification information and/or an identification information relating to the diagnosis and/or analysis apparatus (3) and/or the date of the diagnosis and/or analysis are additionally storable.

5. The cartridge according to any one of the claims 1 to 4, wherein electric, electronic, and/or optical data transfer means (8;11) are provided for reading and/or writing and/or altering data and/or information stored in the storage means (10).

6. The cartridge according to claim 5, wherein the data transfer means comprise the respective connecting means (8) of the cartridge and/or radio frequency transfer means (11).

7. The cartridge according to any one of the claims 1 to 6, wherein the storage means (10) comprise an electrical, magnetical or optical memory member, e. g. EEPROM, FRAM, PROM or battery backed RAM, readable and/or writable via electric or electronic data transfer means or at least one magnetic stripe or an optical memory or a two dimensional barcode.

8. A diagnosis and/or analysis apparatus of a system for point of care diagnosis and/or analysis of body fluids of a patient, comprising:
- electric connecting means (13) connectable to connecting means (8) of a cartridge (2), wherein at least one electrical value each correlating with the concentration of a component of a sample of the patients body fluids to be diagnosed and/or analysed is measurable at the connecting means (8) of the cartridge (2),
- diagnosing and/or analysing means (14) for measuring the respective concentration values via the connecting means (8,13) and evaluating them for determining the concentration of the respective component,
- reading means (15) for reading cartridge specific data and/or information stored in storage means (10) of the cartridge (2),
- wherein the diagnosing and/or analysing means (14) perform the measurement and/or the evaluation of the respective concentration values in accordance to the cartridge specific data and/or information,
wherein the diagnosis and/or analysis apparatus (3) is provided for use with a cartridge (2) having means (9) for measurement of the temperature of the sample,
**characterized in that** an electrical value correlating to the temperature of the probe is measurable at the connecting means (8) of the cartridge (2), wherein the diagnosing and/or analysing means (14) additionally measure the respective temperature value via the connecting means (8,13), wherein the cartridge specific data and/or information comprise calibration data of the respective temperature measurement means (9), wherein the diagnosing and/or analysing means (14) evaluate the respective temperature value by use of appropriate coefficients for determining the probe temperature, wherein the diagnosing and/or analysing means (14) select the appropriate coefficients in accordance to the respective calibration data, and wherein the diagnosing and/or analysing means (14) evaluate the respective concentration value in accordance to the probe temperature.

9. The diagnosis and/or analysis apparatus of claim 8, wherein the diagnosing and/or analysing means (14) evaluate the respective concentration values by use of appropriate coefficients and/or parameters and/or algorithms for determining the concentration of the respective component, and wherein the diagnosing and/or analysing means (14) select the appropriate coefficients and/or parameters and/or algorithms in accordance to the cartridge specific data and/or information.

10. The diagnosis and/or analysis apparatus according to claim 8 or 9, wherein the cartridge specific data and/or information comprises at least one of the following data and/or information:
- calibration and/or characterisation data relating to the respective cartridge (2),
- manufacturing lot data relating to the lot, to which the respective cartridge (2) belongs,
- production date of the respective cartridge (2), shelf life information or expiry date of the respective cartridge (2),
- cartridge type information,
- unique serial number of the respective cartridge (2).

11. The diagnosis and/or analysis apparatus according to any one of the claims 8 to 10, wherein writing means are provided for writing to the storage means (10) of the cartridge (2), wherein processing means are provided for controlling the storing and/or altering of following additional data and/or information.
- in case of a single use cartridge (2) it is stored, whether it is used or not,
- in case of a multiple use cartridge (2) it is stored, how often and/or since when it is used,
- the results of each diagnosis and/or analysis relating to the respective cartridge (2) are stored, wherein a patient identification information and/or an operator identification information and/or an identification information relating to the diagnosis and/or analysis apparatus (3) and/or the date of the diagnosis and/or analysis are additionally storable.

12. The diagnosis and/or analysis apparatus according to any one of the claims 8 to 11, wherein the reading means (15) and/or the writing means cooperate with the storage means (10) via the respective connecting means (8,13) or via radio frequency transfer means (11, 16), for reading and/or storing and/or altering the data and/or information in the storing means (10).

13. The diagnosis and/or analysis apparatus according to any one of the claims 8 to 12, wherein the reading means (15) and/or the writing means are provided for reading and/or writing on and/or altering storage means (10) comprising an electrical memory member, e. g. EEPROM, FRAM, PROM or battery backed RAM, readable and/or writable via electric or electronic data transfer means, or at least one magnetic stripe or an optical memory or a two dimensional barcode.

14. The diagnosis and/or analysis apparatus according to any one of the claims 8 to 13, wherein the diagnosing and/or analyzing means (14) compare the expiry date of the connected cartridge (2) with the current date, and perform the measuring and/or evaluating of the respective values, only if the expiry date is not exceeded.

15. The diagnosis and/or analysis apparatus according to any one of the claims 8 to 14, wherein the diagnosing and/or analysing means (14) compare the serial number of the connected cartridge (2) with invalid serial numbers stored in a memory member (18) of the diagnosis and/or analysis apparatus (3), and perform the measuring and/or evaluating of the respective values, only if the serial number of the connected cartridge (2) is not invalid.

16. The diagnosis and/or analysis apparatus according to any one of the claims 8 to 15, wherein the diagnosing and/or analyzing means (14) in case of a connected single use cartridge (2) check, whether it is used, and in case of a connected multiple use cartridge (2) check, how often and/or since when it is used, and perform the measuring and/or evaluating of the respective values, only if the connected cartridge (2) is still usable.

17. System for point of care diagnosis and/or analysis of body fluids of a patient, comprising :
- a cartridge (2), in particular according to any one of the claims 1 to 7,
- a diagnosis and/or analysis apparatus (3), in particular according to any one of the claims 8 to 15,
- means (6) for measurement the concentration of at least one specific component of a sample of the body fluids,
- connecting means (8, 13) providing communication for electrical values between the cartridge (2) and the diagnosis and/or analysis apparatus (3), wherein said values correlate with at least one of the respective concentrations,
- diagnosing and/or analyzing means (14) measuring the respective concentration value via the connecting means (8,13),
wherein the diagnosing and/or analyzing means (14) evaluate the respective concentration values in accordance to the cartridge specific data and/or information,
**characterized by**:
- storage means (10) for storing cartridge specific data and/or information and
- reading means (15) for reading the data and/or information out of the storage means (10).

## Patentansprüche

1. Kartusche eines Systems zur Diagnose und/oder Analyse von Körperflüssigkeiten eines Patienten am Behandlungsort, die Folgendes umfasst:
- einen Probenaufnahmeraum (4) zur Aufnahme einer Kalibrierflüssigkeit und/oder einer Probe der zu diagnostizierenden und/oder analysierenden Körperflüssigkeiten,
- Mittel (6) zum Messen der Konzentration von mindestens einem bestimmten Bestandteil der Probe,
- elektrische oder optische Anschlussmittel (8), durch die die Kartusche (2) mit einem Diagnose- und/oder Analysegerät (3) verbunden werden kann und an denen ein elektrischer oder optischer Wert gemessen werden kann, der der Konzentration des jeweiligen Bestandteils entspricht,
**gekennzeichnet durch** Speichermittel (10) zum Speichern von kartuschenspezifischen Daten und/oder Informationen, die für das Diagnose- und/oder Analysegerät (3) lesbar sind.

2. Kartusche nach Anspruch 1, wobei die kartuschenspezifischen Daten und/oder Informationen mindestens eine der folgenden Daten und/oder Informationen umfassen:
- Kalibrier- und/oder Kenndaten zu der jeweiligen Kartusche (2),
- Fertigungslosdaten zu dem Los, zu dem die jeweilige Kartusche (2) gehört,
- Herstelldatum der jeweiligen Kartusche (2),
- Haltbarkeitsinformationen oder Verfallsdatum der jeweiligen Kartusche (2),
- Kartuschentypinformationen wie Einweg oder Mehrweg, Anzahl und Art der Messparameter,
- Eindeutige Seriennummer der jeweiligen Kartusche (2).

3. Kartusche nach Anspruch 1 oder 2, wobei die Kartusche (2) Mittel (9) zur Temperaturmessung umfasst, wobei ein elektrischer Wert, der der Temperatur der Probe entspricht, an den Anschlussmitteln (8) messbar ist, wobei die kartuschenspezifischen Daten und/oder Informationen Kalibrierdaten der Temperaturmessmittel umfassen.

4. Kartusche nach einem der Ansprüche 1 bis 3, wobei die Speichermittel (10) so geschaffen werden, dass sie beschreibbar sind, und wobei mindestens eine der folgenden zusätzlichen Daten und/oder Informationen in den Speichermitteln (10) gespeichert oder geändert werden können:
- Im Fall einer Einwegkartusche (2) wird gespeichert, ob sie benutzt wurde oder nicht,
- Im Fall einer Mehrwegkartusche (2) wird gespeichert, wie oft und/oder seit wann sie benutzt wurde,
- Die Ergebnisse jeder Diagnose und/oder Analyse mit Bezug auf die jeweilige Kartusche (2) werden gespeichert, wobei eine Patientenidentifikationsinformation und/oder eine Bedieneridentifikationsinformation und/oder eine Identifikationsinformation zum Diagnose- und/oder Analysegerät (3) und/oder das Datum der Diagnose und/oder Analyse zusätzlich gespeichert werden können.

5. Kartusche nach einem der Ansprüche 1 bis 4, wobei elektrische, elektronische und/oder optische Datenübertragungsmittel (8, 11) zum Lesen und/oder Schreiben und/oder Ändern von in den Speichermitteln (10) gespeicherten Daten und/oder Informationen geschaffen werden.

6. Kartusche nach Anspruch 5, wobei die Datenübertragungsmittel die jeweiligen Anschlussmittel (8) der Kartusche und/oder HF-Übertragungsmittel (11) umfassen.

7. Kartusche nach einem der Ansprüche 1 bis 6, wobei die Speichermittel (10) ein elektrisches, magnetisches oder optisches Speicherbauteil, beispielsweise einen EEPROM, einen FRAM, einen PROM oder einen batteriegepufferten RAM, umfassen, das über elektrische oder elektronische Datenübertragungsmittel oder mindestens einen Magnetstreifen oder einen optischen Speicher oder einen zweidimensionalen Strichcode lesbar und/oder beschreibbar ist.

8. Diagnose- und/oder Analysegerät eines Systems zur Diagnose und/oder Analyse von Körperflüssigkeiten eines Patienten am Behandlungsort, das Folgendes umfasst:
- elektrische Anschlussmittel (13), die mit den Anschlussmitteln (8) einer Kartusche (2) verbunden werden können, wobei mindestens ein elektrischer Wert jeweils der Konzentration eines Bestandteils einer Probe der zu diagnostizierenden und/oder analysierenden Körperflüssigkeiten des Patienten entspricht und an den Anschlussmitteln (8) der Kartusche (2) messbar ist,
- Diagnose- und/oder Analysemittel (14) zum Messen der jeweiligen Konzentrationswerte über die Anschlussmittel (8, 13) und Auswerten zum Ermitteln der Konzentration des jeweiligen Bestandteils,
- Lesemittel (15) zum Lesen von kartuschenspezifischen Daten und/oder Informationen, die in den Speichermitteln (10) der Kartusche (2) gespeichert sind,
wobei die Diagnose- und/oder Analysemittel (14) die Messung und/oder die Auswertung der jeweiligen Konzentrationswerte in Übereinstimmung mit den kartuschenspezifischen Daten und/oder Informationen durchführen,
wobei das Diagnose- und/oder Analysegerät (3) für den Einsatz mit einer Kartusche (2) mit Mitteln (9) zum Messen der Temperatur der Probe geschaffen wird,
**dadurch gekennzeichnet, dass** ein der Temperatur der Probe entsprechender elektrischer Wert an den Anschlussmitteln (8) der Kartusche (2) messbar ist, wobei die Diagnose- und/oder Analysemittel (14) zusätzlich den jeweiligen Temperaturwert über die Anschlussmittel (8, 13) messen, wobei die kartuschenspezifischen Daten und/oder Informationen Kalibrierdaten der jeweiligen Temperaturmessmittel (9) umfassen, wobei die Diagnose- und/oder Analysemittel (14) den jeweiligen Temperaturwert mit Hilfe von geeigneten Koeffizienten zum Ermitteln der Probentemperatur auswerten, wobei die Diagnose- und/oder Analysemittel (14) die geeigneten Koeffizienten in Übereinstimmung mit den jeweiligen Kalibrierdaten auswählen und wobei die Diagnose- und/oder Analysemittel (14) den jeweiligen Konzentrationswert in Übereinstimmung mit der Probentemperatur auswerten.

9. Diagnose- und/oder Analysegerät nach Anspruch 8, wobei die Diagnose- und/oder Analysemittel (14) die jeweiligen Konzentrationswerte mit Hilfe von geeigneten Koeffizienten und/oder Parametern und/oder Algorithmen zum Ermitteln der Konzentration des jeweiligen Bestandteils auswerten und wobei die Diagnose- und/oder Analysemittel (14) die geeigneten Koeffizienten und/oder Parameter und/oder Algorithmen in Übereinstimmung mit den kartuschenspezifischen Daten und/oder Informationen auswählen.

10. Diagnose- und/oder Analysegerät nach Anspruch 8 oder 9, wobei die kartuschenspezifischen Daten und/oder Informationen mindestens eine der folgenden Daten und/oder Informationen umfassen:
- Kalibrier- und/oder Kenndaten zu der jeweiligen Kartusche (2),
- Fertigungslosdaten zu dem Los, zu dem die jeweilige Kartusche (2) gehört,
- Herstelldatum der jeweiligen Kartusche (2),
- Haltbarkeitsinformationen oder Verfallsdatum der jeweiligen Kartusche (2),
- Kartuschentypinformationen,
- Eindeutige Seriennummer der jeweiligen Kartusche (2).

11. Diagnose- und/oder Analysegerät nach einem der Ansprüche 8 bis 10, wobei Schreibmittel zum Schreiben in die Speichermittel (10) der Kartusche (2) geschaffen werden, wobei Verarbeitungsmittel zum Steuern der Speicherung und/oder Änderung der folgenden zusätzlichen Daten und/oder Informationen geschaffen werden:
- Im Fall einer Einwegkartusche (2) wird gespeichert, ob sie benutzt wurde oder nicht,
- Im Fall einer Mehrwegkartusche (2) wird gespeichert, wie oft und/oder seit wann sie benutzt wurde,
- Die Ergebnisse jeder Diagnose und/oder Analyse mit Bezug auf die jeweilige Kartusche (2) werden gespeichert, wobei eine Patientenidentifikationsinformation und/oder eine Bedieneridentifikationsinformation und/oder eine Identifikationsinformation zum Diagnose- und/oder Analysegerät (3) und/oder das Datum der Diagnose und/oder Analyse zusätzlich gespeichert werden können.

12. Diagnose- und/oder Analysegerät nach einem der Ansprüche 8 bis 11, wobei die Lesemittel (15) und/oder die Schreibmittel mit den Speichermitteln (10) zum Lesen und/oder Speichern und/oder Ändern der Daten und/oder Informationen in den Speichermitteln (10) über die jeweiligen Anschlussmittel (8, 13) oder über HF-Übertragungsmittel (11, 16) zusammenarbeiten.

13. Diagnose- und/oder Analysegerät nach einem der Ansprüche 8 bis 12, wobei die Lesemittel (15) und/oder die Schreibmittel zum Lesen und/oder Beschreiben und/oder Ändern der Speichermittel (10) geschaffen werden und ein elektrisches Speicherbauteil, beispielsweise einen EEPROM, einen FRAM, einen PROM oder einen batteriegepufferten RAM umfassen, das über elektrische oder elektronische Datenübertragungsmittel oder mindestens einen Magnetstreifen oder einen optischen Speicher oder einen zweidimensionalen Strichcode gelesen und/oder beschrieben werden kann.

14. Diagnose- und/oder Analysegerät nach einem der Ansprüche 8 bis 13, wobei die Diagnose- und/oder Analysemittel (14) das Verfallsdatum der angeschlossenen Kartusche (2) mit dem aktuellen Datum vergleichen und die Messung und/oder Auswertung der jeweiligen Werte nur durchführen, wenn das Verfallsdatum nicht überschritten ist.

15. Diagnose- und/oder Analysegerät nach einem der Ansprüche 8 bis 14, wobei die Diagnose- und/oder Analysemittel (14) die Seriennummer der angeschlossenen Kartusche (2) mit ungültigen Seriennummern vergleichen, die in einem Speicherbauteil (18) des Diagnose- und/oder Analysegerätes (3) gespeichert sind, und die Messung und/oder Auswertung der jeweiligen Werte nur durchführen, wenn die Seriennummer der angeschlossenen Kartusche (2) nicht ungültig ist.

16. Diagnose- und/oder Analysegerät nach einem der Ansprüche 8 bis 15, wobei die Diagnose- und/oder Analysemittel (14) im Falle einer angeschlossenen Einwegkartusche (2) überprüfen, ob sie benutzt wurde, und im Fall eine angeschlossenen Mehrwegkartusche (2) überprüfen, wie oft und/oder seit wann sie benutzt wurde, und die Messung und/oder Auswertung der jeweiligen Werte nur durchführen, wenn die angeschlossene Kartusche (2) noch benutzbar ist.

17. System zur Diagnose und/oder Analyse von Körperflüssigkeiten eines Patienten am Behandlungsort, das Folgendes umfasst:
- eine Kartusche (2) im Besonderen nach einem der Ansprüche 1 bis 7,
- ein Diagnose- und/oder Analysegerät (3) im Besonderen nach einem der Ansprüche 8 bis 15,
- Mittel (6) zum Messen der Konzentration von mindestens einem bestimmten Bestandteil einer Probe der Körperflüssigkeiten,
- Anschlussmittel (8, 13), die die Kommunikation für elektrische Werte zwischen der Kartusche (2) und dem Diagnose- und/oder Analysegerät (3) herstellen, wobei die genannten Werte mindestens einer der jeweiligen Konzentrationen entsprechen,
- Diagnose- und/oder Analysemittel (14), die den jeweiligen Konzentrationswert über die Anschlussmittel (8, 13) messen,
wobei die Diagnose- und/oder Analysemittel (14) die jeweiligen Konzentrationswerte in Übereinstimmung mit den kartuschenspezifischen Daten und/oder Informationen auswerten, **gekennzeichnet durch**:
- Speichermittel (10) zum Speichern von kartuschenspezifischen Daten und/oder Informationen und
- Lesemittel (15) zum Lesen der Daten und/oder Informationen aus den Speichermitteln (10).

## Revendications

1. Cartouche d'un système pour diagnostic et/ou analyse de fluides corporels d'un patient sur le site d'administration des soins, comprenant :
- un espace de réception d'échantillon (4) pour recevoir un fluide d'étalonnage et/ou un échantillon des fluides corporels à diagnostiquer et/ou analyser,
- un moyen (6) pour mesurer la concentration d'au moins un composant spécifique de l'échantillon,
- un moyen de connexion électrique ou optique (8), avec lequel la cartouche (2) peut être connectée à un appareil de diagnostic et/ou d'analyse (3), et auquel une valeur électrique ou optique peut être mesurée, qui est en corrélation avec la concentration du composant respectif,
**caractérisé par** un moyen de stockage (10) pour stocker des données et/ou informations spécifiques à la cartouche, qui peuvent être lues par l'appareil de diagnostic et/ou d'analyse (3).

2. Cartouche selon la revendication 1, dans laquelle les données et/ou informations spécifiques à la cartouche comprennent au moins une des données et/ou informations suivantes :
- données d'étalonnage et/ou de caractérisation relative à la cartouche respective (2),
- données de lot de fabrication relatives au lot auquel la cartouche respective (2) appartient,
- date de production de la cartouche respective (2),
- information de durée limite de stockage ou date de péremption de la cartouche respective (2),
- informations à propos du type de cartouche, comme utilisation unique /multiple, nombre et type de paramètres de mesure,
- numéro de série unique de la cartouche respective (2).

3. Cartouche selon la revendication 1 ou 2, la cartouche (2) comprenant un moyen (9) de mesure de température, où une valeur électrique en corrélation avec la température de l'échantillon est mesurable au moyen de connexion (8), où les données et/ou informations spécifiques à la cartouche comprennent des données d'étalonnage du moyen de mesure de température.

4. Cartouche selon une quelconque des revendications 1 à 3, dans laquelle le moyen de stockage (10) est prévu pour être inscriptible, et dans laquelle au moins une des données et/ou informations additionnelles suivantes est stockable ou modifiable dans le moyen de stockage (10) :
- dans le cas d'une cartouche (2) à utilisation unique, le fait qu'elle a été utilisée ou non est enregistré,
- dans le cas d'une cartouche (2) à usages multiples, le nombre d'utilisations est enregistré, et/ou depuis quand elle est en utilisation,
- les résultats de chaque diagnostic et/ou analyse relatifs à la cartouche respective (2) sont stockés, où une information d'identification de patient et/ou une information d'identification d'opérateur et/ou une information d'identification relative à l'appareil de diagnostic et/ou d'analyse (3) et/ou la date de diagnostic et/ou d'analyse sont additionnellement enregistrables.

5. Cartouche selon une quelconque des revendications 1 à 4, où un moyen de transfert électrique, électronique et/ou optique (8, 11) est prévu pour lire et/ou écrire et/ou modifier des données et/ou informations stockées dans le moyen de stockage (10).

6. Cartouche selon la revendication 5, où le moyen de transfert de données comprend le moyen de connexion respectif (8) de la cartouche et/ou un moyen de transfert par fréquence radio (11).

7. Cartouche selon une quelconque des revendications 1 à 6, où le moyen de stockage (10) comprend un élément mémoire électrique, magnétique ou optique, par exemple une mémoire EEPROM, FRAM, PROM ou RAM alimentée par pile, qui peut être lu et/ou écrit par un moyen électrique ou électronique de transfert de données, ou au moins une piste magnétique ou une mémoire optique ou un code à barres à deux dimensions.

8. Appareil de diagnostic et/ou d'analyse d'un système pour diagnostic et/ou analyse de fluides corporels d'un patient sur le site d'administration des soins, comprenant :
- un moyen de connexion électrique (13) qui peut être connecté au moyen de connexion (8) d'une cartouche (2), où au moins une valeur électrique, chacune en corrélation avec la concentration d'un composant d'un échantillon des fluides corporels de patient à diagnostiquer et/ou analyser, est mesurable au moyen de connexion (8) de la cartouche (2),
- un moyen de diagnostic et/ou d'analyse (14) pour mesurer les valeurs de concentration respectives via les moyens de connexion (8, 13) et les évaluer pour déterminer la concentration du composant respectif,
- un moyen de lecture (15) pour lire des données et/ou informations spécifiques à la cartouche stockées dans le moyen de stockage (10) de la cartouche (2),
où le moyen de diagnostic et/ou d'analyse (14) effectue la mesure et/ou l'évaluation des valeurs de concentration respectives en fonction des données et/ou informations spécifiques à la cartouche,
où l'appareil de diagnostic et/ou d'analyse (3) est prévu pour utilisation avec une cartouche (2) qui a un moyen (9) pour mesurer la température de l'échantillon, **caractérisé en ce qu'**une valeur électrique en corrélation avec la température de la sonde est mesurable au moyen de connexion (8) de la cartouche (2), où le moyen de diagnostic et/ou d'analyse (14) mesure additionnellement la valeur de température respective via les moyens de connexion (8, 13), où les données et/ou informations spécifiques à la cartouche comprennent des données d'étalonnage du moyen de mesure de température respectif (9), où le moyen de diagnostic et/ou d'analyse (14) évalue la valeur de température respective par utilisation de coefficients appropriés pour déterminer la température de sonde, où le moyen de diagnostic et/ou d'analyse (14) sélectionne les coefficients appropriés en fonction des données d'étalonnage respectives, et où le moyen de diagnostic et/ou d'analyse (14) évalue la valeur de concentration respective en fonction de la température de sonde.

9. Appareil de diagnostic et/ou d'analyse selon la revendication 8, dans lequel le moyen de diagnostic et/ou d'analyse (14) évalue les valeurs de concentration respectives par l'utilisation de coefficients et/ou paramètres et/ou algorithmes appropriés pour déterminer la concentration du composant respectif, et où le moyen de diagnostic et/ou d'analyse (14) sélectionne les coefficients et/ou paramètres et/ou algorithmes appropriés en fonction des données et/ou informations spécifiques à la cartouche.

10. Appareil de diagnostic et/ou d'analyse selon la revendication 8 ou 9, dans lequel les données et/ou informations spécifiques à la cartouche comprennent au moins une des données et/ou informations suivantes :
- données d'étalonnage et/ou de caractérisation relatives à la cartouche respective (2),
- données de lot de fabrication relatives au lot auquel la cartouche respective (2) appartient,
- date de production de la cartouche respective (2),
- information de durée limite de stockage ou date de péremption de la cartouche respective (2),
- informations de type de cartouche,
- numéro de série unique de la cartouche respective (2).

11. Appareil de diagnostic et/ou d'analyse selon une quelconque des revendications 8 à 10, dans lequel un moyen d'écriture est prévu pour écrire sur le moyen de stockage (10) de la cartouche (2), dans lequel un moyen de traitement est prévu pour commander le stockage et/ou la modification de données et/ou informations additionnelles suivantes :
- dans le cas d'une cartouche (2) à utilisation unique, le fait qu'elle a été utilisée ou non est enregistré,
- dans le cas d'une cartouche (2) à usages multiples, le nombre d'utilisations est enregistré, et/ou depuis quand elle est en utilisation,
- les résultats de chaque diagnostic et/ou analyse relatifs à la cartouche respective (2) sont enregistrés, où une information d'identification de patient et/ou une information d'identification d'opérateur et/ou une information d'identification relative à l'appareil de diagnostic et/ou d'analyse (3) et/ou la date de diagnostic et/ou d'analyse peuvent additionnellement être stockées.

12. Appareil de diagnostic et/ou d'analyse selon une quelconque des revendications 8 à 11, dans lequel le moyen de lecture (15) et/ou le moyen d'écriture coopère avec le moyen de stockage (10) via les moyens de connexion respectifs (8, 13) ou via des moyens de transfert par fréquence radio (11, 16) pour lire et/ou stocker et/ou modifier les données et/ou informations dans le moyen de stockage (10).

13. Appareil de diagnostic et/ou d'analyse selon une quelconque des revendications 8 à 12, dans lequel le moyen de lecture (15) et/ou le moyen d'écriture sont prévus pour lire et/ou écrire et/ou modifier le moyen de stockage (10) qui comprend un élément mémoire électrique, par exemple une mémoire EEPROM, FRAM, PROM ou RAM alimentée par pile, qui peut être lu et/ou écrit via un moyen de transfert de données électrique ou électronique, ou au moins une piste magnétique ou une mémoire optique ou un code à barres à deux dimensions.

14. Appareil de diagnostic et/ou d'analyse selon une quelconque des revendications 8 à 13, dans lequel le moyen de diagnostic et/ou d'analyse (14) compare la date de péremption de la cartouche connectée (2) avec la date courante, et n'effectue la mesure et/ou n'évalue les valeurs respectives que si la date de péremption n'est pas dépassée.

15. Appareil de diagnostic et/ou d'analyse selon une quelconque des revendications 8 à 14, dans lequel le moyen de diagnostic et/ou d'analyse (14) compare le numéro de série de la cartouche (2) connectée avec des numéros de série invalides stockés dans un élément formant mémoire (18) de l'appareil de diagnostic et/ou d'analyse (3), et n'effectue la mesure et/ou n'évalue les valeurs respectives que si le numéro de série de la cartouche (2) connectée n'est pas invalide.

16. Appareil de diagnostic et/ou d'analyse selon une quelconque des revendications 8 à 15, dans lequel le moyen de diagnostic et/ou d'analyse (14) vérifie, dans le cas d'une cartouche connectée (2) à utilisation unique, si elle a été utilisée, et dans le cas d'une cartouche connectée (2) à utilisations multiples, combien de fois elle a été utilisée ou depuis quand, et n'effectue les mesures et/ou n'évalue les valeurs respectives que si la cartouche connectée (2) est encore utilisable.

17. Système pour diagnostic et/ou analyse de fluides corporels d'un patient sur le site d'administration des soins, comprenant :
- une cartouche (2), en particulier selon une quelconque des revendications 1 à 7,
- un appareil de diagnostic et/ou d'analyse (3), en particulier selon une quelconque des revendications 8 à 15,
- un moyen (6) pour mesurer la concentration d'au moins un composant spécifique d'un échantillon des fluides corporels,
- des moyens de connexion (8, 13) qui procurent une communication entre la cartouche (2) et l'appareil de diagnostic et/ou d'analyse (3) pour des valeurs électriques, où lesdites valeurs sont en corrélation avec au moins une des concentrations respectives,
- un moyen de diagnostic et/ou d'analyse (14) qui mesure la valeur de concentration respective via les moyens de connexion (8, 13),
dans lequel le moyen de diagnostic et/ou d'analyse (14) évalue les valeurs de concentration respectives en fonction des données et/ou informations spécifiques à la cartouche,
**caractérisé par** :
- un moyen de stockage (10) pour stocker des données et/ou informations spécifiques à la cartouche et
- un moyen de lecture (15) pour lire les données et/ou informations à partir du moyen de stockage (10).
